# EUROPEAN PATENT SPECIFICATION

(11) **EP 3 476 376 B1**
(45) Date of publication and mention of the grant of the patent: **25.03.2020**
(21) Application number: 16906302.1
(22) Date of filing: 23.06.2016
(51) Int. Cl.: A61F 13/15, B65H 21/02, B65H 23/04, B65H 23/188, B65H 39/14, A61F 13/514

(54) **METHOD AND DEVICE FOR MANUFACTURING SHEET-LIKE MEMBER OF ABSORBENT ARTICLE**
VERFAHREN UND VORRICHTUNG ZUR HERSTELLUNG EINES BAHNFÖRMIGEN ELEMENTS EINES SAUGFÄHIGEN ARTIKELS
PROCÉDÉ ET DISPOSITIF DE FABRICATION D'ÉLÉMENT DE TYPE FEUILLE D'ARTICLE ABSORBANT

(43) Date of publication of application: 01.05.2019
(73) Proprietor: Unicharm Corporation, Shikokuchuo-shi, Ehime 799-0111 (JP)
(72) Inventor: NAKANO, Takumi, Kanonji-shi Kagawa 769-1602 (JP); HIKIMOTO, Mitsuhiko, Kanonji-shi Kagawa 769-1602 (JP); YAMAMOTO, Hiroki, Kanonji-shi Kagawa 769-1602 (JP)
(74) Representative: Staeger & Sperling Partnerschaftsgesellschaft mbB
(86) International application number: PCT/JP2016/068705
(87) International publication number: WO 2017/221385

(56) References cited:
- EP-A1- 0 957 867
- EP-A1- 1 199 057
- EP-A1- 1 314 411
- WO-A1-01/56525
- JP-A- 2002 087 665
- JP-A- 2002 512 566
- JP-A- 2003 521 339
- JP-A- 2006 515 251
- JP-B2- 3 351 763

## Description

### Technical Field

The present invention relates to a method and an apparatus for manufacturing a sheet-shaped member of an absorbent article.

### Background Art

Sometimes an image such as a pattern or text is rendered at a predetermined position on an absorbent article such as a disposable diaper. In the manufacture of such absorbent articles, if a printing device is installed in-line on a manufacturing line so as to print sheets synchronized with manufacture of the absorbent articles, the manufacturing line may be contaminated by ink solvent, or the scale and cost of equipment may increase. A roll body configured by a sheet pre-printed with images at a prescribed pitch and wound into a roll shape is accordingly employed to supply an image-rendered sheet from the roll body (see Patent Literature 1, 2).

### Citation List

### Patent Document

[Patent Document 1] Japanese Patent No.4246020
[Patent Document 1] Japanese Patent No.3351763

### Summary of Invention

### Technical Problem

Winding a sheet in a roll body configuration at a uniform tension from the start of winding to the end of winding is difficult. The pitch of images accordingly varies as the sheet is being dispensed from the wound roll. In the manufacturing method described in Patent Literature 1, a prescribed pattern is accordingly pre-printed at a print pitch shorter than a cut length of a continuous member. The cut length and the print pitch are then aligned by stretching the continuous member as it is being dispensed, and by controlling the speed at which the continuous member is dispensed based on any difference in position between the position where the pattern should be positioned on a cut member and the position where the pattern is actually positioned on the continuous member as it is being conveyed, so that the pattern is adjusted to be positioned at a predetermined location thereon.

However, in the manufacturing method described in Patent Literature 1, the position of the pattern on the conveyed continuous member is detected immediately after the continuous member has been dispensed (immediately after being supplied from the roll body). There is accordingly a long distance from the detection position to a bonding position where the continuous member and a separate sheet are bonded. As a result, there is a concern in the manufacturing method described in Patent Literature 1 that the position of the pattern might shift partway through conveying due to fluctuations in the tension of the continuous member.

In the manufacturing method described in Patent Literature 2, an adjustment means is disposed at a location near to a bonding position. The print pitch of the pattern is then matched to the cut length of the absorbent article by adjusting the rotation speed of the adjustment means, thereby controlling so as to position the pattern at a predetermined location on the absorbent article. The sort of problems that arise with the manufacturing method described in Patent Literature 1 (problems caused by there being a long distance from the detection position to the bonding position) therefore do not occur with the manufacturing method described in Patent Literature 2. However, the adjustment means in Patent Literature 2 needs to adjust both a feed speed of the sheet and the position of the pattern. In other words, the adjustment means needs to perform both pattern pitch adjustment and pattern phase adjustment. Such dual adjustment makes hunting liable to occur when the rotation speed of the adjustment means is changing, and the possibility of an unstable state in terms of sheet tension and the like arises.

An objective of the invention is to both adjust the feed speed of an image-rendered sheet configuring absorbent articles and adjust the position of the images in a stable manner.

### Solution to Problem

A primary aspect of the invention to achieve the above objective is a method for manufacturing a sheet-shaped member by superimposing a continuous first sheet and a continuous second sheet at a merge position to configure the sheet-shaped member having contiguously arranged absorbent articles, the manufacturing method including:
a supply process of supplying the first sheet having extensibility and formed periodically with an image from a roll body;
a feed process of feeding the first sheet toward the merge position by controlling a feed speed of the first sheet such that an amount of the first sheet equivalent to a single unit of the absorbent article is fed every time an amount of the sheet-shaped member equivalent to a single unit of the absorbent article is conveyed; and
a position adjustment process of adjusting a position of the image with respect to the absorbent article by detecting the image on the first sheet, and changing a path length from a feed position where the feed speed of the first sheet is controlled to the merge position based on a result of detecting the image.

Other features of the present invention will become apparent from the present description and the attached drawings.

### Advantageous Effects of Invention

According to the present invention, to both adjust the feed speed of an image-rendered sheet configuring absorbent articles and adjust the position of the images in a stable manner can be achieved.

### Brief Description of Drawings

Fig. 1A is a schematic plan view of an absorbent article 10. Fig. 1B is a schematic cross-section sectioned along B-B in Fig. 1A.
Fig. 2 is an explanatory diagram of a first embodiment of a manufacturing system 100 for the absorbent article 10.
Fig. 3 is a schematic plan view of a continuous body 10A (sheet-shaped member) of the absorbent articles 10.
Fig. 4 is a schematic plan view of a first sheet 14A.
Fig. 5 is an explanatory diagram of a modified example of the first embodiment of a manufacturing system 100 for the absorbent article 10.
Fig. 6 is an explanatory diagram of a second embodiment of a manufacturing system 100 for the absorbent article 10.
Fig. 7 is an explanatory diagram of a first modified example of the second embodiment.
Fig. 8 is an explanatory diagram of a second modified example thereof.
Fig. 9 is an explanatory diagram of a third modified example thereof.
Fig. 10A to Fig. 10C are explanatory diagrams of a third embodiment of a manufacturing system 100 for the absorbent article 10.
Fig. 11A is a diagram to explain a positional relationship at a joint in the first sheet 14A.
Fig. 11B is a diagram to explain the first sheet 14A after joining.

### Description of Embodiments

At least the following matters are made clear from the present description and the attached drawings.

A method for manufacturing a sheet-shaped member by superimposing a continuous first sheet and a continuous second sheet at a merge position to configure the sheet-shaped member having contiguously arranged absorbent articles will become clear, the manufacturing method including: a supply process of supplying the first sheet having extensibility and formed periodically with an image from a roll body; a feed process of feeding the first sheet toward the merge position by controlling a feed speed of the first sheet such that an amount of the first sheet equivalent to a single unit of the absorbent article is fed every time an amount of the sheet-shaped member equivalent to a single unit of the absorbent article is conveyed; and a position adjustment process of adjusting a position of the image with respect to the absorbent article by detecting the image on the first sheet, and changing a path length from a feed position where the feed speed of the first sheet is controlled to the merge position based on a result of detecting the image. Such a manufacturing method enables both the feed speed of the first sheet (image-rendered sheet) configuring absorbent articles to be adjusted and the position of the images to be adjusted in a stable manner.

Preferably, in the feed process, detecting the image on the first sheet, and controlling the feed speed of the first sheet based on a result of detecting the image. This enables the feed speed of the first sheet to be detected by utilizing the images formed on the first sheet.

It is preferable to further include a separate feed process of feeding the first sheet toward the merge position by controlling a feed speed of the first sheet such that an amount of the first sheet equivalent to a single unit of the absorbent article is fed every time an amount of the sheet-shaped member equivalent to a single unit of the absorbent article is conveyed, the separate feed process being performed between the feed position and a position where the path length is changed. This facilitates feeding of the first sheet toward the merge position while the feed speed is maintained in a synchronized state.

A position where the path length is changed is preferably disposed closer to the merge position than to the feed position. This enables a higher response speed for adjusting the position of the images.

It is preferable to further include a turn process of turning a conveyance direction of the first sheet by wrapping the first sheet around a bar-shaped member having an axial direction inclined with respect to a direction orthogonal to the conveyance direction of the first sheet, wherein the feed position is arranged at the conveyance direction downstream side of the bar-shaped member. This enables the effects of resistance from the bar-shaped member to be suppressed.

It is preferable to further include a separate feed process of feeding the first sheet toward the bar-shaped member by controlling the feed speed of the first sheet such that an amount of the first sheet equivalent to a single unit of the absorbent article is fed every time an amount of the sheet-shaped member equivalent to a single unit of the absorbent article is conveyed, the separate feed process being performed at the conveying direction upstream side of the bar-shaped member. This enables the effects of resistance from the bar-shaped member to be suppressed.

In the supply process, when splicing a waiting second roll body onto a first roll body currently being supplied so as to supply a first sheet from the second roll body after supply of a first sheet from the first roll body, preferably a position of a start point on the first roll body of one of the absorbent articles is aligned with a position of a start point on the second roll body of one of the absorbent articles, and the first sheet of the first roll body and the first sheet of the second roll body are spliced together. This means that there is no sudden change to the path length due to the effects of the joint in the first sheet.

An apparatus for manufacturing a sheet-shaped member by superimposing a continuous first sheet and a continuous second sheet at a merge position to configure the sheet-shaped member having contiguously arranged absorbent articles will become clear, the manufacturing apparatus including: a supply unit configured to supply the first sheet having extensibility and formed periodically with an image, from a roll body; a feed unit configured to feed the first sheet toward the merge position by controlling a feed speed of the first sheet such that an amount of the first sheet equivalent to a single unit of the absorbent article is fed every time an amount of the sheet-shaped member equivalent to a single unit of the absorbent article is conveyed; and a position adjustment unit configured to adjust a position of the image with respect to the absorbent article by detecting the image on the first sheet and changing a path length from a feed position to the merge position based on a result of detecting the image, between a feed position where the feed speed of the first sheet is controlled and the merge position. Such a manufacturing apparatus enables both the feed speed of the first sheet (image-rendered sheet) configuring absorbent articles to be adjusted and the position of images to be adjusted in a stable manner.

### ===First Embodiment===

A basic structure of an absorbent article 10 will first be described, and then a manufacturing system 100 for the absorbent article 10 will be described.

### <Basic Structure of Absorbent Article 10>

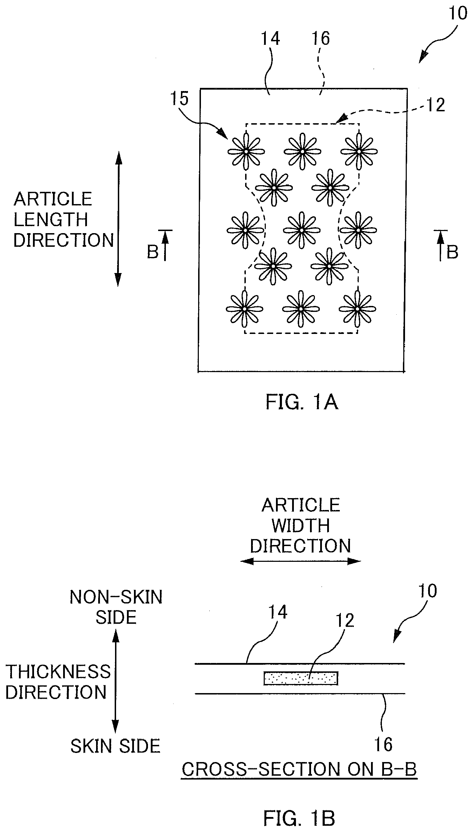

Fig. 1A is a schematic plan view of the absorbent article 10. Fig. 1B is a schematic cross-section sectioned along B-B in Fig. 1A.

In the description that follows, a side that should be positioned on the skin side of a wearer is sometimes referred to as the "skin side", and a side that should be positioned away from the skin side of the wearer is sometimes referred to as the "non-skin side" . Moreover, as illustrated in Fig. 1A, a length direction of the elongated absorbent article 10 is sometimes referred to as the "article length direction", and the width direction of the elongated absorbent article 10 is sometimes referred to as the "article width direction". Moreover, as illustrated in Fig. 1B, a direction orthogonal to the article length direction and the article width direction is sometimes referred to as the "thickness direction".

The absorbent article 10 is an article employed to absorb and retain excreted fluid (such as urine) of the wearer. The absorbent article 10 of the present embodiment is more specifically a disposable diaper. The absorbent article 10 is, however, not limited to being a disposable diaper, and may, for example, be a sanitary napkin or the like. The absorbent article 10 may be configured by additionally attaching a fastening tape or the like to a configuration as illustrated in Fig. 1A and Fig. 1B.

The absorbent article 10 includes an absorbent body 12, a back sheet 14, and a top sheet 16 (see Fig. 1B). The absorbent body 12 is a member employed to absorb and retain liquid (excreted fluid). The back sheet 14 is a non-liquid permeable sheet member disposed on the non-skin side of the absorbent body 12. The top sheet 16 is a liquid permeable sheet member disposed on the skin side of the absorbent body 12.

In the absorbent article 10 of the present embodiment, an image 15 configured from prescribed text, a pattern, a cartoon character, or the like is printed on the back sheet 14 (see Fig. 1A). The design quality of the external appearance of the absorbent article 10 is improved by the image 15. The image 15 also enables a user to visually ascertain information related to the absorbent article 10 (for example a trade name and orientation of the absorbent article 10).

### <Manufacturing System 100 for the Absorbent Article 10>

### • Overall Configuration

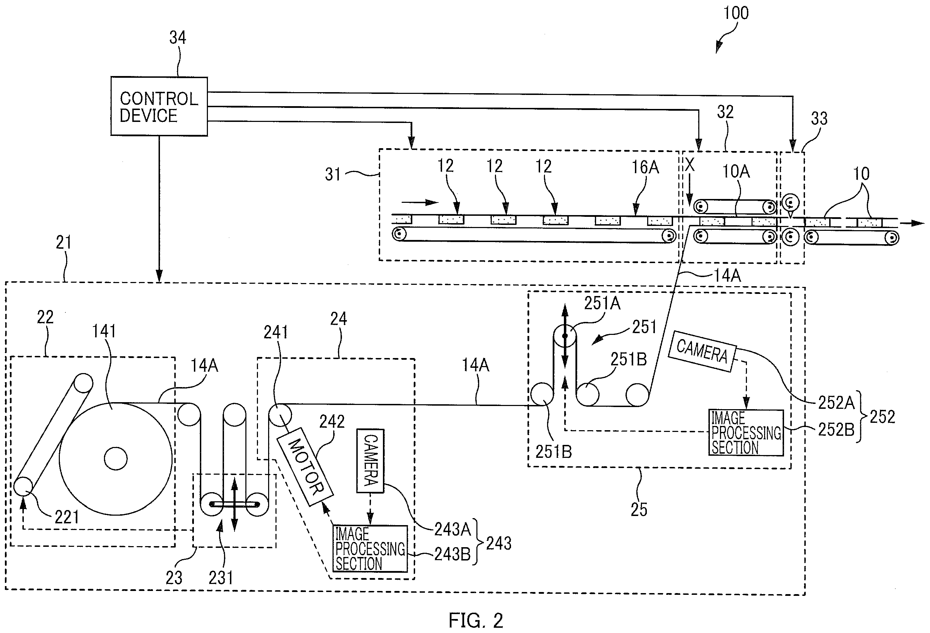

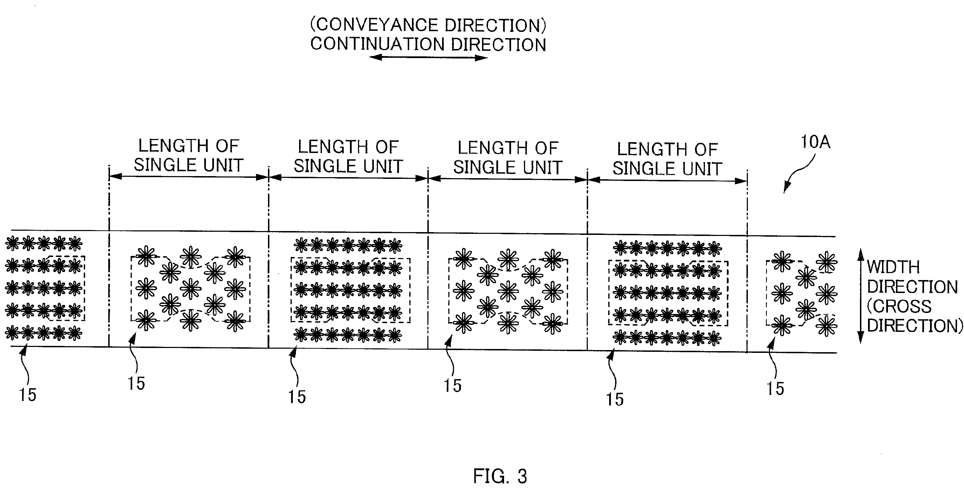

Fig. 2 is an explanatory diagram of the first embodiment of the manufacturing system 100 for the absorbent article 10. Fig. 3 is a schematic plan view of a continuous body 10A (sheet-shaped member) of the absorbent articles 10.

The manufacturing system 100 of the first embodiment is a manufacturing apparatus (manufacturing system) for manufacturing the absorbent article 10. The manufacturing system 100 includes a first conveying device 21, a second conveying device 31, a joining device 32, a cutting device 33, and a control device 34 (see Fig. 2). The continuous body 10A (see Fig. 3) of the absorbent articles 10 is produced by superimposing a first sheet 14A conveyed by the first conveying device 21 and a second sheet 16A conveyed by the second conveying device 31 at a merge position (a position marked by X in the drawing) in the joining device 32. The produced continuous body 10A is then cut at a prescribed pitch (a length of a single unit of the absorbent article 10) in the cutting device 33 so as to manufacture the absorbent article 10.

The first conveying device 21 is a device to convey the first sheet 14A that will become the back sheet 14 to the joining device 32 (to the merge position). The first conveying device 21 is described in detail later.

The second conveying device 31 is a device to convey the second sheet 16A that will become the top sheet 16 to the joining device 32. The second sheet 16A is a continuous sheet-shaped member that will become the top sheet 16. The second conveying device 31 conveys the second sheet 16A together with absorbent bodies 12. The absorbent bodies 12 are fixed to the second sheet 16A at a prescribed pitch in a row along the conveyance direction. The second conveying device 31 includes a belt conveyor, conveyor rollers, or the like as a conveying mechanism to convey the second sheet 16A. The second conveying device 31 employed here to convey the second sheet 16A in a state affixed with the absorbent bodies 12 is preferably provided with a suction belt conveyor including a suction function as a conveying mechanism to convey such a second sheet 16A.

The joining device 32 is a device employed to superimpose the first sheet 14A and the second sheet 16A at the merge position and join them together. The joining device 32 includes, for example, a pair of upper and lower belt conveyors as a conveying mechanism to convey the first sheet 14A and the second sheet 16A in a superimposed state. The first sheet 14A and the second sheet 16A are conveyed in a state sandwiched between the pair of upper and lower belt conveyors. The first sheet 14A and the second sheet 16A are compressed by the belt conveyors as they are being conveyed, and are joined together by a hot-melt adhesive or the like. The continuous body 10A of the absorbent articles 10 (see Fig. 3) is formed by superimposing the first sheet 14A and the second sheet 16A in the joining device 32.

In the continuous body 10A of the absorbent articles 10, the absorbent articles 10 are disposed at the prescribed pitch in a row contiguously along the conveyance direction. The continuous body 10A is a sheet-shaped member configured by the absorbent articles 10 contiguously arranged in a row. Note that in Fig. 3, the absorbent articles 10 are arranged in a row such that the article length direction (see Fig. 1A) is aligned with the length direction of the continuous body 10A (the conveyance direction of the continuous body 10A). However, absorbent articles 10 may be arranged in a row such that the article width direction (see Fig. 1A) is aligned with the length direction of the continuous body 10A (the conveyance direction of the continuous body 10A) . The pitch of the row of the absorbent articles 10 in the continuous body 10A is equivalent to the length of a single unit of the absorbent article 10.

The cutting device 33 is a device employed to cut the continuous body 10A of the absorbent articles 10 at the prescribed pitch. The cutting device 33 includes, for example, a cutter roll and an anvil roll as a mechanism for cutting the continuous body 10A. The cutting device 33 rotates the cutter roll and the anvil roll so as to cut the continuous body 10A of the absorbent articles 10 at the prescribed pitch while the continuous body 10A is being conveyed along the conveyance direction.

The control device 34 is a controller to control the first conveying device 21, the second conveying device 31, the joining device 32, and the cutting device 33. In particular, the control device 34 controls the conveyance speeds of each device (the first conveying device 21, the second conveying device 31, the joining device 32, and the cutting device 33) so as to synchronize their conveyance speeds. Namely, the control device 34 controls the conveyance speeds of each of the devices to match the speed at which a length of a single unit of the absorbent article 10 is being conveyed. Although the conveyance speeds of each of the devices are synchronized, this does not necessarily mean that the conveyance speeds of each of the devices are the same as each other, since the tension imparted to the sheets when conveyed by each of the devices is different (the sheet length of a single unit of the absorbent article 10 is different in each of the devices).

### • First conveying device 21

The first conveying device 21 is a device for conveying the first sheet 14A that will become the back sheet 14 to the joining device 32. The first sheet 14A is a continuous sheet-shaped member that will become the back sheet 14. The first conveying device 21 includes a supply unit 22, a tension regulation unit 23, a feed unit 24, and a position adjustment unit 25.

The supply unit 22 is a unit (device) to supply the first sheet 14A from a sheet roll 141. The supply unit 22 includes a drive section 221 to rotate the sheet roll 141. The sheet roll 141 is a member configured by winding the continuous first sheet 14A into a roll shape. The supply unit 22 drives the drive section 221 according to instructions from the control device 34 (and the tension regulation unit 23), thereby supplying the first sheet 14A from the sheet roll 141.

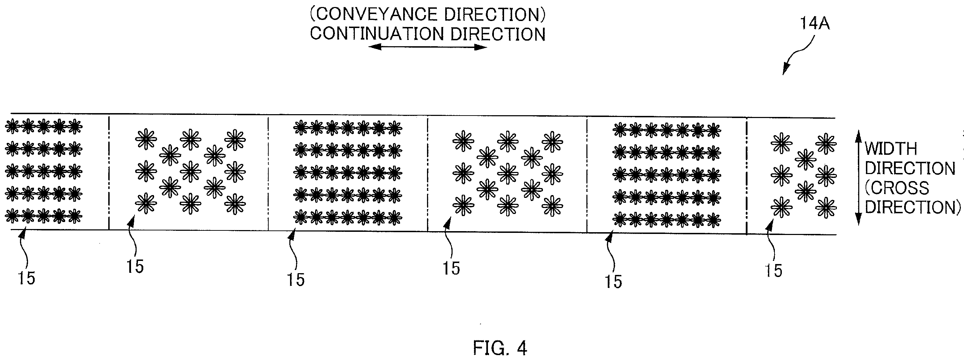

Fig. 4 is a schematic plan view of the first sheet 14A. The first sheet 14A is a continuous sheet having extensibility, and is periodically formed with the images 15. There may be plural types of the image 15 formed on the first sheet 14A, or there may be a single type thereof. Although the image 15 in this example is a pattern, the image 15 may be configured by text, a cartoon character, or the like.

The images 15 are arranged in a row along the first sheet 14A at the prescribed pitch (the length of a single unit of the absorbent article 10) in the conveyance direction. The pitch of the images 15 (the spacing between the images 15) is equivalent to the length of a single unit of the absorbent article 10. Note that the pitch of the images 15 (the length of a single unit of the absorbent article 10) changes according to the tension imparted to the first sheet 14A. When making the sheet roll 141 it is difficult to wind the first sheet 14A at a uniform tension from the start of winding to the end of winding. The pitch of the images 15 on the first sheet 14A is accordingly sometimes not constant when in the wound state wound into the sheet roll 141, and instead varies slightly.

The tension regulation unit 23 is a unit (device) to regulate the tension on the first sheet 14A. The tension regulation unit 23 includes dancer rolls 231, and the tension imparted to the first sheet 14A is regulated by movement of the dancer rolls 231. The tension regulation unit 23 includes a detection section (not illustrated in the drawings) to detect the position of the dancer rolls 231. The drive section 221 of the supply unit 22 increases or decreases the rotation speed of the sheet roll 141 according to the results detected for the position of the dancer rolls 231 so as to stabilize the tension on the first sheet 14A. Although there are two of the dancer rolls 231 illustrated in the drawings, there may be any number of two or more of the dancer rolls 231. The tension regulation unit 23 is disposed between the supply unit 22 and the feed unit 24. This means that the first sheet 14A supplied to the feed unit 24 is a sheet that has been regulated to a prescribed tension by the tension regulation unit 23.

Although the tension regulation unit 23 of the first embodiment includes the dancer rolls 231, there is no limitation to such a configuration as long as the tension on the first sheet 14A can be regulated. However, as described below, due to being equipped with the dancer rolls 231, the tension regulation unit 23 is able to exhibit a storage function by accumulating the first sheet 14A.

The feed unit 24 is a unit (device) to control the feed speed of the first sheet 14A. The feed unit 24 feeds the first sheet 14A toward the merge position at a speed synchronized to the conveyance speed at the merge position. Namely, the feed unit 24 feeds an amount of the first sheet 14A equivalent to a single unit of the absorbent article 10 toward the merge position every time an amount of the continuous body 10A equivalent to a single unit of the absorbent article 10 is conveyed at the merge position. In cases in which the tension on the first sheet 14A (the continuous body 10A) at the merge position is different from the tension on the first sheet 14A at the feed unit 24, the length of an amount equivalent to a single unit of the absorbent article 10 at the merge position is different to the length of an amount equivalent to a single unit of the absorbent article 10 at the feed unit 24. Thus even when synchronized, the conveyance speed at the merge position may be a different speed to the feed speed of the feed unit 24.

The feed unit 24 includes a feed roll 241 and a feed motor 242. The first sheet 14A is fed by the feed motor 242 rotating the feed roll 241. The feed unit 24 drives the feed motor 242 according to a target speed (synchronization speed) instructed by the control device 34, so as to control the feed speed of the first sheet 14A. The position of the feed roll 241 is a position where the feed speed of the first sheet 14A is controlled (feed position).

The feed unit 24 includes a speed detection section 243. The speed detection section 243 detects the feed speed of the first sheet 14A, and is a section to control (perform feedback control on) the feed speed of the first sheet 14A based on the results of detection. The speed detection section 243 includes, for example, a camera 243A and an image processing section 243B, and uses the camera 243A to capture the images 15 formed on the first sheet 14A. The speed detection section 243 then detects the feed speed of the first sheet 14A by analyzing image data of the camera 243A (results of detecting the images 15) using the image processing section 243B. The feed unit 24 then, based on any difference between the speed detected by the speed detection section 243 and the target speed, performs feedback control on the feed motor 242 so as to control the feed speed of the first sheet 14A such that the feed speed of the first sheet 14Abecomes the target speed (synchronization speed) . Thus in the present embodiment, the images 15 on the first sheet 14A are detected, and the feed speed of the first sheet 14A is controlled based on the results of detecting the images 15. The images 15 formed on the first sheet 14A are accordingly utilized to enable the feed speed of the first sheet 14A to be detected. The method by which the feed speed of the first sheet 14A is detected is, however, not limited thereto. For example, the feed speed of the first sheet 14A may be detected by detecting the rotation speed of the feed roll 241. The present embodiment, however, is able to directly detect the feed speed of the first sheet 14A with better precision than if the feed speed of the first sheet 14A was to be detected based on the rotation speed of the feed roll 241.

The position adjustment unit 25 is a unit (device) to adjust the position of the images 15 with respect to the absorbent articles 10 by changing a path length from the feed position (the position of the feed roll 241) to the merge position.

The position adjustment unit 25 includes, as a path length changing mechanism 251, a movable roll 251A, a pair of following rolls 251B, and a moving mechanism (not illustrated in the drawings) to move the movable roll 251A. The movable roll 251A is disposed between the pair of following rolls 251B, and the first sheet 14A is wrapped around the movable roll 251A. The path length is lengthened when the movable roll 251A moves in a direction away from the following rolls 251B. The path length is shortened when the movable roll 251A moves in a direction to approach the following rolls 251B.

The position adjustment unit 25 includes a position detection section 252. The position detection section 252 detects the position of the images 15 on the first sheet 14A, and then changes the path length (moves the movable roll 251A) based on the results of detection. For example, the position detection section 252 includes a camera 252A and an image processing section 252B, and uses the camera 252A to capture (perform synchronization capture) of the images 15 on the first sheet 14A synchronized to the conveyance speed (synchronization speed) of the continuous body 10A at the merge position. The image data (results of detecting the images 15) from the camera 252A are analyzed by the image processing section 252B to detect the position of the images 15 on the first sheet 14A. The image capture cycle of the camera 252A is the cycle over which an amount of the continuous body 10A equivalent to a single unit of the absorbent article 10 is conveyed at the merge position (or an integer multiple of such a cycle) . If the detected position of the images 15 on the first sheet 14A is offset toward the conveyance direction downstream side (toward the merge position), the position adjustment unit 25 lengthens the path length to adjust the position of the images 15. Moreover, if the detected position of the images 15 on the first sheet 14A is offset toward the conveyance direction upstream side, the position adjustment unit 25 shortens the path length to adjust the position of the images 15.

The position detection section 252 detects the position of the images 15 on the first sheet 14A at the upstream side of the merge position (see Fig. 2). This enables the position of the images 15 on the first sheet 14A to be adjusted prior to superimposing the first sheet 14A and the second sheet 16A. The position detection section 252 may, however, detect the position of the images 15 on the first sheet 14A at the downstream side of the merge position. Namely, the position detection section 252 may detect the position of the images 15 on the first sheet 14A configuring the continuous body 10A when the first sheet 14A and the second sheet 16A are in a superimposed state at the downstream side of the merge position. This also enables the position of the images 15 to be adjusted with respect to the absorbent article 10.

Under conditions in which the position adjustment unit 25 is being continuously operated, there is only a slight difference between the path length based on the result of detecting the position of a given image 15 and the path length based on the result of detecting the position of the next image 15 (i.e. only a slight amount of change in path length) . There is also only an extremely small amount of change in path length with respect to the distance from the feed position (the position of the feed roll 241) to the merge position, and therefore extension or contraction of the first sheet 14A accompanying the change in path length is tolerable. The effect of extension or contraction of the first sheet 14A accompanying such change in path length is eliminated at the stage when the section of the first sheet 14A that was present on the path when the path length changed has been conveyed to the merge position, and so there is no accumulated effect. The section of the first sheet 14A that is present on the path from the feed position (the position of the feed roll 241) to the merge position is in a tension imparted state so as not to sag, and this is accordingly a state in which the first sheet 14A is slightly stretched compared to a state in which tension has not been imparted. This enables extension or contraction of the first sheet 14A accompanying the change in path length to be absorbed by elastic deformation of the first sheet 14A on the path.

However, as already described, the feed unit 24 feeds an amount of the first sheet 14A equivalent to a single unit of the absorbent article 10 toward the merge position every time an amount of the continuous body 10A equivalent to a single unit of the absorbent article 10 is conveyed at the merge position. In other words, the feed unit 24 adjusts the feed speed of the first sheet 14A so as to be synchronized to the conveyance speed of the continuous body 10A at the merge position. At the merge position, however, not only does the speed of the first sheet 14A need to have been adjusted so as to be synchronized with the conveyance speed of the continuous body 10A, but the position of the images 15 on the first sheet 14A also needs to be adjusted so that the position of the images 15 does not shift with respect to the absorbent article 10. Suppose that the position of the images 15 on the first sheet 14A were to be adjusted at the feed unit 24, then due to the distance from the feed roll 241 (feed position) to the merge position being long in such cases, the position of the images 15 might shift while the first sheet 14A is being conveyed due to fluctuations in the tension on the first sheet 14A. In contrast thereto, in the present embodiment, the position adjustment unit 25 is disposed at the downstream side of the feed position, and the position of the images 15 with respect to the absorbent article 10 is adjusted by the position adjustment unit 25. The position of the images 15 on the first sheet 14A can accordingly be adjusted at the merge position in a state not liable to be affected by fluctuations in the tension on the first sheet 14A.

In the present embodiment, the feed unit 24 adjusts the feed speed of the first sheet 14A, and the position adjustment unit 25 adjusts the position of the images 15 on the first sheet 14A. The adjustment of the feed speed of the first sheet 14A and the adjustment of the position of the images 15 on the first sheet 14A are accordingly performed separately in this manner. This enables the position adjustment unit 25 to adjust the position of the images 15 on the first sheet 14A while the conveyance speed of the first sheet 14A is being maintained. The present embodiment is accordingly less prone to hunting occurring than cases in which the adjustment to the feed speed of the first sheet 14A and the adjustment of the position of the images 15 on the first sheet 14A are performed by the same adjustment means. This thereby enables the first sheet 14A to be conveyed stably.

The position adjustment unit 25 in the present embodiment is also disposed nearer to the merge position than the position of the feed unit 24 (feed position). In other words, the position where the path length is changed in the present embodiment is disposed nearer to the merge position than the position of the feed unit 24 (feed position). This enables a higher response speed from changing the path length to adjusting the position of the images 15 on the first sheet 14A at the merge position.

### <Modified Example of First Embodiment>

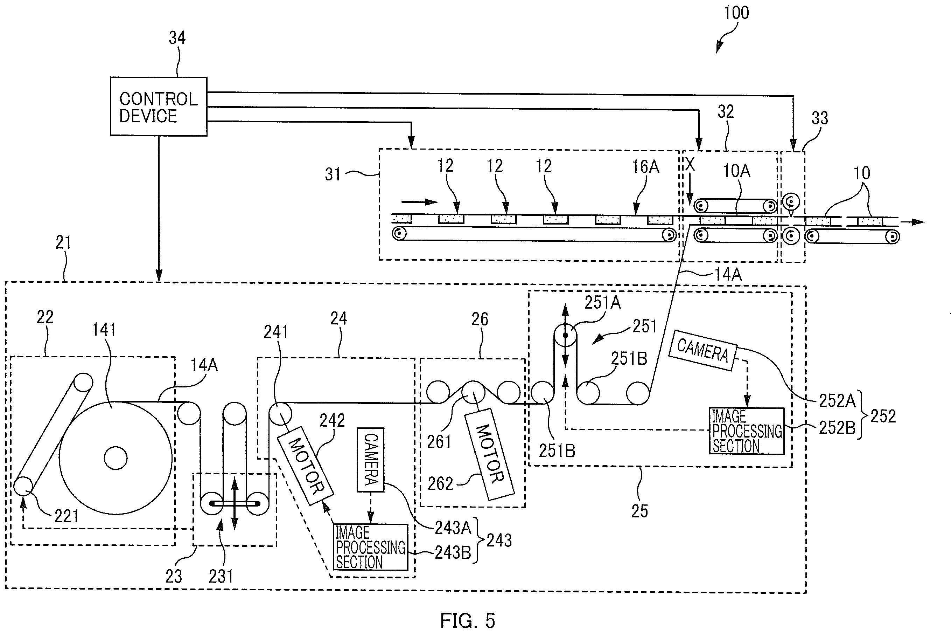

Fig. 5 is an explanatory diagram of a modified example of the first embodiment of the manufacturing system 100 for the absorbent article 10. The modified example of the manufacturing system 100 includes an auxiliary feed unit 26 added to a first conveying device 21 in addition to that of the manufacturing system 100 of Fig. 2.

The auxiliary feed unit 26 is a unit (device) to feed the first sheet 14A at a prescribed feed speed. The auxiliary feed unit 26 feeds the first sheet 14A toward the merge position at a speed synchronized to the conveyance speed at the merge position. Namely, the auxiliary feed unit 26 feeds an amount of the first sheet 14A equivalent to a single unit of the absorbent article 10 toward the merge position every time an amount of the continuous body 10A equivalent to a single unit of the absorbent article 10 is conveyed at the merge position. The length equivalent to a single unit of the absorbent article 10 is different at the upstream side of the auxiliary feed unit 26 to at the downstream side thereof in cases in which the tension on the first sheet 14A is different at the upstream side of the auxiliary feed unit 26 to at the downstream side thereof. This means that even when the feed speed of the feed unit 24 and the feed speed of the auxiliary feed unit 26 are synchronized, the speeds may be different from each other.

The auxiliary feed unit 26 includes an auxiliary feed roll 261 and an auxiliary feed motor 262. The first sheet 14A is fed by the auxiliary feed motor 262 rotating the auxiliary feed roll 261. The auxiliary feed unit 26 drives the auxiliary feed motor according to a target speed (synchronization speed) instructed by the control device 34, so as to control the feed speed of the first sheet 14A.

The auxiliary feed unit 26 is disposed between the feed unit 24 and the position adjustment unit 25. In other words, the auxiliary feed unit 26 is disposed between the feed position of the feed unit 24 (the position of the feed roll 241) and the path length change position of the position adjustment unit 25 (the position of the path length changing mechanism 251) . This enables the first sheet 14A, which is being fed at a speed synchronized by the feed unit 24, to be fed toward the merge position while this synchronized state is maintained.

### ===Second Embodiment===

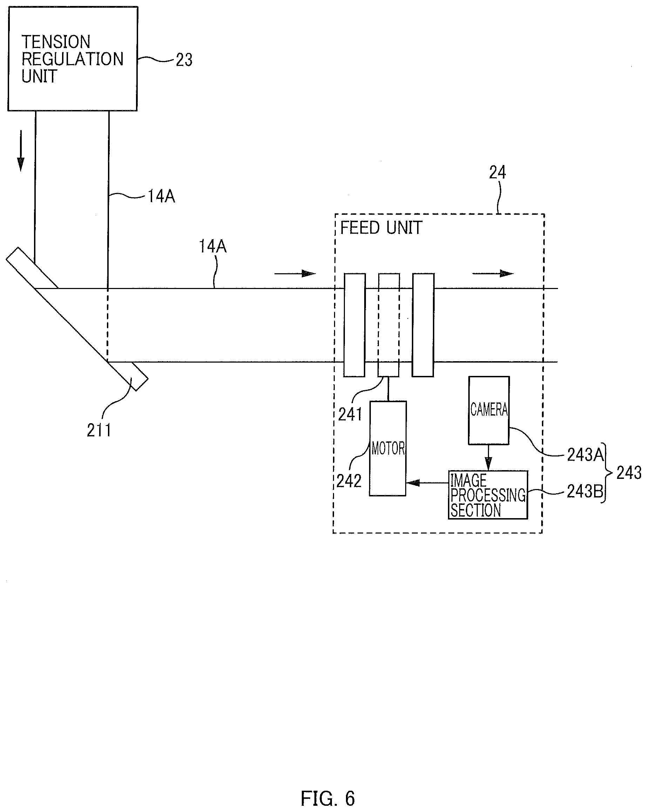

Fig. 6 is an explanatory diagram of a second embodiment of a manufacturing system 100 for an absorbent article 10. The configuration of a tension regulation unit 23 of the second embodiment is similar to that of the first embodiment. The configuration of a feed unit 24 of the second embodiment is similar to that of the first embodiment. Moreover, similarly to in the manufacturing system 100 of the first embodiment illustrated in Fig. 2, a supply unit 22 is also provided at the upstream side of the tension regulation unit 23 in the conveyance direction. The configuration at the downstream side of the illustrated feed unit 24 in the conveyance direction is similar to that of the manufacturing system 100 of the first embodiment illustrated in Fig. 2. Images 15 formed on the first sheet 14A are omitted from illustration in Fig. 6.

A turn bar 211 (bar-shaped member) is provided in the second embodiment between the tension regulation unit 23 and the feed unit 24. The turn bar 211 is a round bar-shaped member, and is a member employed to turn the conveyance direction of the first sheet 14A. The turn bar 211 is disposed with an axial direction inclined with respect to a direction orthogonal to the conveyance direction of the first sheet 14A (i.e. with respect to the width direction of the first sheet 14A). The conveyance direction of the first sheet 14A is turned by the first sheet 14A being wrapped around the turn bar 211 disposed inclined in such a manner. Provision of the turn bar 211 enables a more compact first conveying device 21 (a shorter manufacturing line) to be achieved, and enables an increase in the degrees of freedom for the conveyance path of the first sheet 14A. The turn bar 211 is preferably disposed so as to be fixed.

The feed unit 24 in the second embodiment is disposed at the downstream side of the turn bar 211 in the conveyance direction. This means that even if there is some resistance imparted by the turn bar 211 to conveyance of the first sheet 14A, as long as the first sheet 14A is fed from the feed unit 24 at a synchronized feed speed (synchronized speed), then at the downstream side of the feed unit 24 in the conveyance direction, the first sheet 14A can still be fed toward the merge position while maintaining this synchronized state. Suppose the turn bar 211 were to be disposed at the downstream side of the feed unit 24 in the conveyance direction, then the first sheet 14A would receive resistance from the turn bar 211, and this might reduce the synchronization precision. The feed unit 24 is accordingly preferably disposed at the downstream side of the turn bar 211 in the conveyance direction in cases in which there is a turn bar 211 installed in the manufacturing system 100.

### <Modified Example of Second Embodiment>

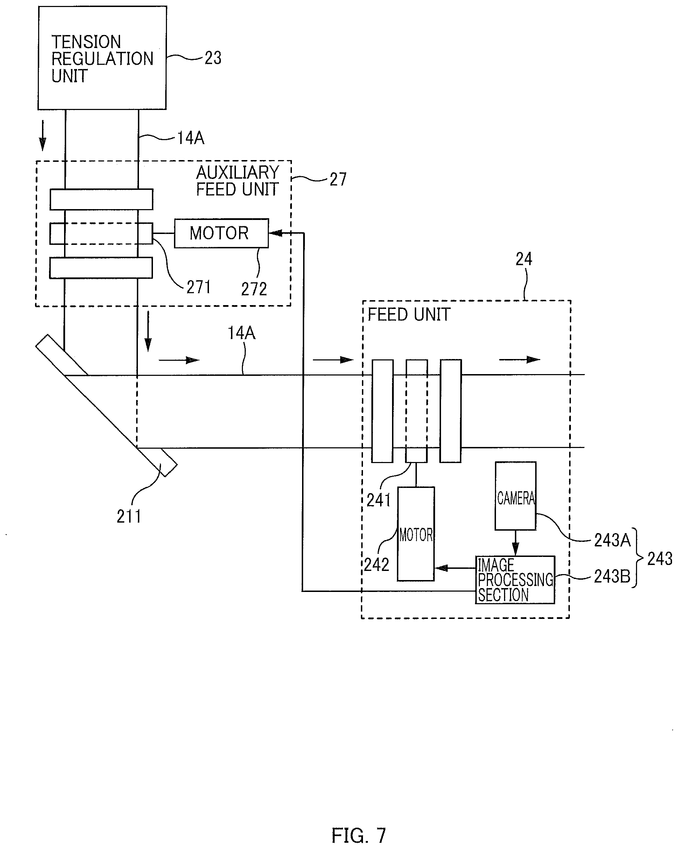

Fig. 7 is an explanatory diagram of a first modified example of the second embodiment. In the first modified example, an auxiliary feed unit 27 is added at the upstream side of a turn bar 211 in the conveyance direction compared to that of Fig. 6.

The auxiliary feed unit 27 is a unit (device) to feed the first sheet 14A at a prescribed feed speed. The auxiliary feed unit 27 feeds the first sheet 14A toward the turn bar 211 at a speed synchronized to the feed speed of the feed unit 24. Namely, the auxiliary feed unit 27 feeds an amount of the first sheet 14A equivalent to a single unit of the absorbent article 10 toward the merge position every time an amount of the continuous body 10A equivalent to a single unit of the absorbent article 10 is conveyed at the merge position. The feed speed of the first sheet 14A by the auxiliary feed unit 27 may be the same as, or different from, the feed speed of the first sheet 14A by the feed unit 24.

The auxiliary feed unit 27 includes an auxiliary feed roll 271 and an auxiliary feed motor 272. The first sheet 14A is fed by the auxiliary feed motor 272 rotating the auxiliary feed roll 271. The auxiliary feed unit 27 drives the auxiliary feed motor 272 based on results of detection by a speed detection section 243 of the feed unit 24, so as to control the feed speed of the first sheet 14A. The method of controlling the auxiliary feed unit 27 is not limited thereto.

Similarly to in the second embodiment illustrated in Fig. 6, the feed unit 24 in the first modified example is disposed at the downstream side of the turn bar 211 in the conveyance direction. This means that even if resistance is imparted by the turn bar 211 to conveyance of the first sheet 14A, the first sheet 14A can still be fed toward the merge position while the synchronized state is maintained.

The auxiliary feed unit 27 is disposed in the first modified example at the upstream side of the turn bar 211 in the conveyance direction. In the first modified example, at both the conveyance direction upstream side and the downstream side of the turn bar 211, an amount of the first sheet 14A equivalent to a single unit of the absorbent article 10 is fed every time an amount of the continuous body 10A equivalent to a single unit of the absorbent article 10 is conveyed. This enables the effects of resistance at the turn bar 211 to be reduced.

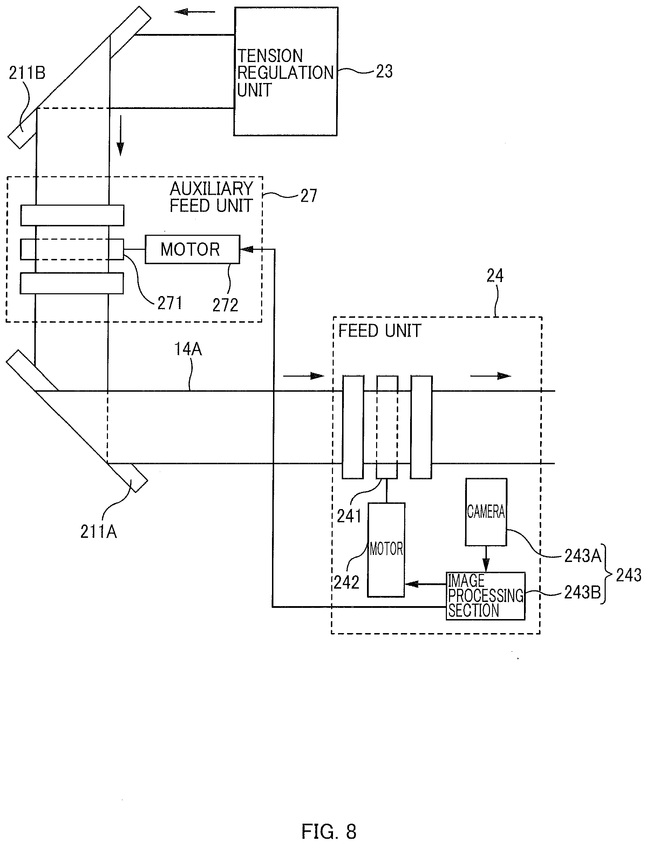

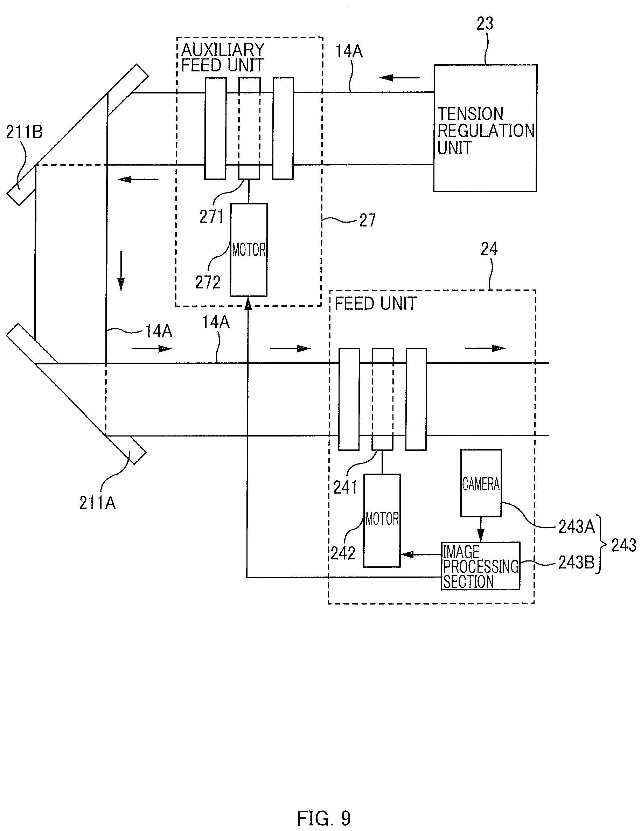

Fig. 8 is an explanatory diagram of a second modified example. Fig. 9 is an explanatory diagram of a third modified example. As illustrated in the second and third modified examples, plural turn bars 211 (211A, 211B) may be provided between the tension regulation unit 23 and the feed unit 24. Note that although there are two of the turn bars 211 illustrated in Fig. 8 and Fig. 9, any number of two or more of the turn bars 211 may be provided.

In cases in which plural of the turn bars 211 are provided, the feed unit 24 is preferably disposed at the conveyance direction downstream side of a turn bar 211A positioned furthest downstream in the conveyance direction, as illustrated in Fig. 8 and Fig. 9. This enables the first sheet 14A to be fed toward the merge position while the synchronized state is maintained, even when resistance to conveyance of the first sheet 14A is imparted by the turn bar 211A.

In cases in which plural of the turn bar 211 are provided, preferably the auxiliary feed unit 27 is disposed at the conveyance direction upstream side of the turn bar 211A positioned furthest downstream in the conveyance direction (see Fig. 8 and Fig. 9), similarly to in the first modified example. However, a configuration lacking an auxiliary feed unit 27 may be adopted when there are plural turn bars 211 provided.

As illustrated in Fig. 8, the auxiliary feed unit 27 may be arranged so as to be adjacent at the upstream side of the conveyance direction furthest downstream turn bar 211A, so that the turn bar 211A positioned furthest downstream in the conveyance direction is interposed between the auxiliary feed unit 27 and the feed unit 24. This enables the effects of resistance at the conveyance direction furthest downstream turn bar 211A to be reduced.

Alternatively, as illustrated in Fig. 9, the auxiliary feed unit 27 may be disposed at the conveyance direction upstream side of a turn bar 211B positioned furthest upstream in the conveyance direction. This enables the plural turn bars 211 to be disposed so as to be interposed between the feed unit 24 and the auxiliary feed unit 27, enabling the effects of resistance at the plural turn bars 211 to be reduced.

### ===Third Embodiment===

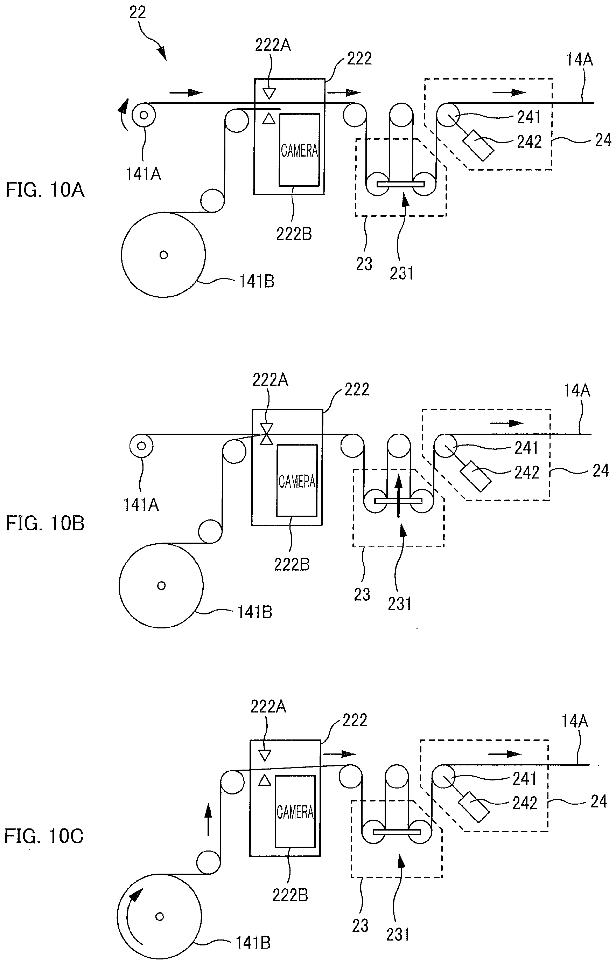

Fig. 10A to Fig. 10C are explanatory diagrams of a third embodiment of a manufacturing system 100 for the absorbent article 10. A splicing device 222 is provided to the supply unit 22 of the manufacturing system 100 of the third embodiment. Units other than the supply unit 22 are similar to those of the manufacturing system 100 of the first embodiment illustrated in Fig. 2. However, dancer rolls 231 of a tension regulation unit 23 of the third embodiment also function as a storage mechanism to accumulate the first sheet 14A.

The splicing device 222 is a device to splice a waiting second sheet roll 141B onto a first sheet roll 141A currently being supplied. The splicing device 222 includes a splicing mechanism 222A and a position detection section 222B. The splicing mechanism 222A is a mechanism to join the first sheet 14A of the second sheet roll 141B to the first sheet 14A of the first sheet roll 141A. The splicing mechanism 222A may include a cutting mechanism to cut the first sheet 14A, as required. The position detection section 222B detects the position of the images 15 formed on the first sheet 14A.

Fig. 10A illustrates a state in which the supply unit 22 is supplying the first sheet 14A from the first sheet roll 141A. The first sheet 14A supplied from the first sheet roll 141A is accumulated in the tension regulation unit 23.

When the first sheet 14A that is being supplied from the first sheet roll 141A has almost run out, the supply unit 22 uses the splicing device 222 to splice the waiting second sheet roll 141B onto the first sheet roll 141A currently being supplied, as illustrated in Fig. 10B. The splicing device 222 cuts the first sheet 14A of the first sheet roll 141A, as required, and joins a trailing end portion of the first sheet 14A of the first sheet roll 141A and a leading end portion of the first sheet 14A of the second sheet roll 141B together (the first sheet 14A is spliced) . The conveyance of the first sheet 14A (the first sheet 14A of the first sheet roll 141A) at the conveyance direction upstream side of the tension regulation unit 23 is stopped while this is being performed. However, the tension regulation unit 23 moves the dancer rolls 231 for adjusting the tension on the first sheet 14A, and the section of the first sheet 14A that has been accumulated in the tension regulation unit 23 thereby continues to be supplied to the feed unit 24. This enables the first sheet 14A to be spliced without stopping the action to feed the first sheet 14A from the feed unit 24. Alternatively, the first sheet 14A may be spliced after stopping the action to feed the first sheet 14A from the feed unit 24. Adopting such an approach means that the tension regulation unit 23 does not need to be provided with a storage function to accumulate the first sheet 14A.

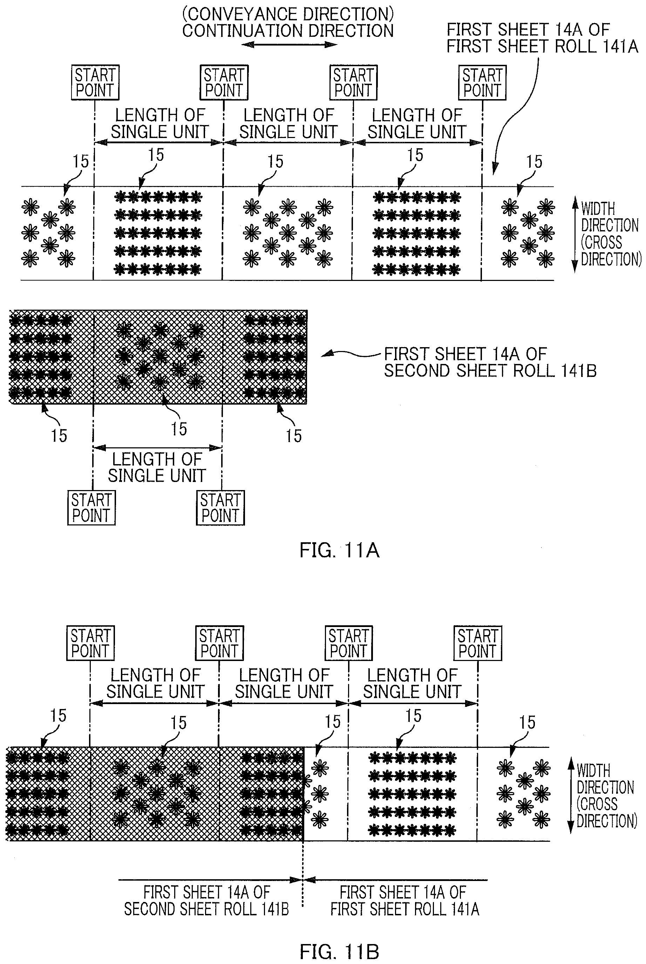

Fig. 11A is a diagram to explain a positional relationship at a joint in the first sheet 14A. Fig. 11B is a diagram to explain the first sheet 14A after joining. For ease of explanation, shading is applied to the first sheet 14A of the second sheet roll 141B. The first sheet 14A of the second sheet roll 141B (the first sheet 14A at the lower side in Fig. 11A) is set in advance such that a start point position of a single unit of the absorbent article 10 is at a predetermined position.

The position detection section 222B of the splicing device 222 detects positions where the images 15 are formed on the first sheet 14A of the first sheet roll 141A (the first sheet 14A at the upper side in Fig. 11A), and detects the positions of start points of single units of the absorbent article 10. Based on the position detection results of the position detection section 222B, the supply unit 22 then positions a given start point on the first sheet 14A of the first sheet roll 141A at the predetermined position, and stops the conveyance of the first sheet 14A from the first sheet roll 141A at this position (from this point in time the first sheet 14A that is supplied to the feed unit 24 is the section of the first sheet 14A that was accumulated in the tension regulation unit 23). Based on the position detection results of the position detection section 222B, the supply unit 22 then aligns the position of the first sheet 14A of the first sheet roll 141A such that a position of a start point on the first sheet 14A of the first sheet roll 141A is aligned in position with the start point position of the first sheet 14A on the second sheet roll 141B set in advance.

Instead of being set in advance such that the start point position is at a predetermined position, the first sheet 14A of the second sheet roll 141B (the first sheet 14A at the lower side in Fig. 11A) may, similarly to with the first sheet roll 141A, be conveyed based on position detection results of the position detection section of the splicing device 222 such that a given start point position on the first sheet 14A of the second sheet roll 141B is at a predetermined position (a position aligned with a start point position on the first sheet 14A of the first sheet roll 141A). However, in such cases a position detection section (a separate position detection section to the position detection section 222B) is required to detect the position of the images 15 formed on the first sheet 14A of the second sheet roll 141B.

The splicing device 222 then joins the first sheet 14A of the first sheet roll 141A and the first sheet 14A of the second sheet roll 141B together in a state in which the start point positions on both sheets are aligned. As long as a state is achieved in which the start point positions on both sheets are aligned, the position where the sheets are joined together (the adhered position) need not be at the position of a start point. As illustrated in Fig. 11B, the images 15 are arranged on the first sheet 14A after joining at a prescribed pitch (the length of a single unit of the absorbent article 10) in a row along the conveyance direction.

After the first sheet 14A of the second sheet roll 141B has been joined, the supply unit 22 supplies the first sheet 14A from the second sheet roll 141B, as illustrated in Fig. 10C. The first sheet 14A can accordingly be continuously supplied in this manner by equipping the supply unit 22 with the splicing device 222.

In the present embodiment, when the first sheet 14A is supplied from the second sheet roll 141B, the joint is fed out from the feed unit 24 so as to reach the position adjustment unit 25. At this point in time, the position adjustment unit 25 detects the image 15 at the joint of the first sheet 14A (see Fig. 11B), and adjusts the path length based on the detection result. In the present embodiment, as illustrated in Fig. 11B, the images 15 are arranged in a row along the conveyance direction at the prescribed pitch (the length of a single unit of the absorbent article 10) even at the joint. This means that the position adjustment unit 25 does not change the path length suddenly even when the path length is adjusted based on the detection results of the images 15 at the joint. This enables the first sheet 14A to be conveyed stably in the present embodiment.

### ===Other===

The embodiments described above are merely to facilitate understanding of the invention, and are not meant to be interpreted in a manner limiting the scope of the invention. The invention can of course be modified and improved without departing from the gist thereof and the invention includes functional equivalents of such modifications and improvements.

### Reference Signs List

- 10:: absorbent article
- 10A:: continuous body of absorbent articles
- 12:: absorbent body
- 14:: back sheet
- 14A:: first sheet
- 141:: sheet roll
- 141A:: first sheet roll
- 141B:: second sheet roll
- 15:: image
- 16:: top sheet
- 16A:: second sheet
- 21:: first conveying device
- 211:: turn bar
- 22:: supply unit
- 221:: drive section
- 222:: splicing device
- 222A:: splicing mechanism
- 222B:: position detection section
- 23:: tension regulation unit
- 231:: dancer rolls
- 24:: feed unit
- 241:: feed roll
- 242:: feed motor
- 243:: speed detection section
- 243A:: camera
- 243B:: image processing section
- 25:: position adjustment unit
- 251:: path length changing mechanism
- 251A:: movable roll
- 251B:: following roll
- 252:: position detection section
- 252A:: camera
- 252B:: image processing section
- 26:: auxiliary feed unit
- 261:: auxiliary feed roll
- 262:: feed motor
- 27:: auxiliary feed unit
- 271:: auxiliary feed roll
- 272:: feed motor
- 31:: second conveying device
- 32:: joining device
- 33:: cutting device
- 34:: control device
- 100:: manufacturing system

## Claims

1. A method for manufacturing a sheet-shaped member by superimposing a continuous first sheet and a continuous second sheet at a merge position to configure the sheet-shaped member having contiguously arranged absorbent articles, the manufacturing method comprising:
a supply process of supplying the first sheet having extensibility and formed periodically with an image from a roll body;
a feed process of feeding the first sheet toward the merge position by controlling a feed speed of the first sheet such that an amount of the first sheet equivalent to a single unit of the absorbent article is fed every time an amount of the sheet-shaped member equivalent to a single unit of the absorbent article is conveyed; and
a position adjustment process of adjusting a position of the image with respect to the absorbent article by detecting the image on the first sheet, and changing a path length from a feed position where the feed speed of the first sheet is controlled to the merge position based on a result of detecting the image.

2. The sheet-shaped member manufacturing method according to claim 1, wherein
in the feed process, detecting the image on the first sheet, and controlling the feed speed of the first sheet based on a result of detecting the image.

3. The sheet-shaped member manufacturing method according to claim 1 or claim 2, further comprising a separate feed process of feeding the first sheet toward the merge position by controlling a feed speed of the first sheet such that an amount of the first sheet equivalent to a single unit of the absorbent article is fed every time an amount of the sheet-shaped member equivalent to a single unit of the absorbent article is conveyed, the separate feed process being performed between the feed position and a position where the path length is changed.

4. The sheet-shaped member manufacturing method according to any one of claim 1 to claim 3, wherein a position where the path length is changed is disposed closer to the merge position than to the feed position.

5. The sheet-shaped member manufacturing method according to any one of claim 1 to claim 4, further comprising a turn process of turning a conveyance direction of the first sheet by wrapping the first sheet around a bar-shaped member having an axial direction inclined with respect to a direction orthogonal to the conveyance direction of the first sheet, wherein
the feed position is arranged at the conveyance direction downstream side of the bar-shaped member.

6. The sheet-shaped member manufacturing method according to claim 5, further comprising a separate feed process of feeding the first sheet toward the bar-shaped member by controlling the feed speed of the first sheet such that an amount of the first sheet equivalent to a single unit of the absorbent article is fed every time an amount of the sheet-shaped member equivalent to a single unit of the absorbent article is conveyed, the separate feed process being performed at the conveying direction upstream side of the bar-shaped member.

7. The sheet-shaped member manufacturing method according to any one of claim 1 to claim 6, wherein
in the supply process, when splicing a waiting second roll body onto a first roll body currently being supplied so as to supply a first sheet from the second roll body after supply of a first sheet from the first roll body,
a position of a start point on the first roll body of one of the absorbent articles is aligned with a position of a start point on the second roll body of one of the absorbent articles, and the first sheet of the first roll body and the first sheet of the second roll body are spliced together.

8. An apparatus for manufacturing a sheet-shaped member by superimposing a continuous first sheet and a continuous second sheet at a merge position to configure the sheet-shaped member having contiguously arranged absorbent articles, the manufacturing apparatus comprising:
a supply unit configured to supply the first sheet having extensibility and formed periodically with an image, from a roll body;
a feed unit configured to feed the first sheet toward the merge position by controlling a feed speed of the first sheet such that an amount of the first sheet equivalent to a single unit of the absorbent article is fed every time an amount of the sheet-shaped member equivalent to a single unit of the absorbent article is conveyed; and
a position adjustment unit configured to adjust a position of the image with respect to the absorbent article by detecting the image on the first sheet and changing a path length from a feed position to the merge position based on a result of detecting the image, between a feed position where the feed speed of the first sheet is controlled and the merge position.

## Patentansprüche

1. Herstellungsverfahren eines lagenförmigen Elements durch Übereinanderlegen einer kontinuierlichen ersten Lage und einer kontinuierlichen zweiten Lage an einer Zusammenführungsposition, um das lagenförmige Element mit angrenzend angeordneten absorbierenden Artikeln auszubilden, das Herstellungsverfahren umfassend:
einen Bereitstellungsprozess zum Bereitstellen der ersten Lage, die Dehnbarkeit aufweist und periodisch mit einem Bild von einem Walzenkörper ausgebildet ist; und
einen Zuführprozess zum Zuführen der ersten Lage in Richtung der Zusammenführungsposition durch Steuerung einer Zuführgeschwindigkeit der ersten Lage, so dass eine Menge der ersten Lage, die einer einzelnen Einheit des absorbierenden Artikels entspricht, jedes Mal zugeführt wird, wenn eine Menge des lagenförmigen Elements, die einer einzelnen Einheit des absorbierenden Artikels entspricht, gefördert wird; und
einen Positionsanpassungsvorgang zum Anpassen einer Position des Bildes in Bezug auf den absorbierenden Artikel durch Erfassen des Bildes auf der ersten Lage und zum Ändern einer Pfadlänge von einer Zuführposition, bei der die Zuführgeschwindigkeit der ersten Lage gesteuert wird, zu der Zusammenführungsposition basierend auf einem Ergebnis der Erfassung des Bildes.

2. Herstellungsverfahren des lagenförmigen Elements gemäß Anspruch 1, wobei beim Zuführprozess, Erfassung des Bildes auf der ersten Lage und Steuerung der Zuführgeschwindigkeit der ersten Lage basierend auf dem Ergebnis der Erfassung des Bildes.

3. Herstellungsverfahren des lagenförmigen Elements gemäß Anspruch 1 oder Anspruch 2, das ferner einen getrennten Zuführprozess zum Zuführen der ersten Lage in Richtung der Zusammenführungsposition umfasst, durch Steuerung einer Zufuhrgeschwindigkeit der ersten Lage, so dass eine Menge der ersten Lage, die einer einzelnen Einheit des absorbierenden Artikels entspricht, jedes Mal zugeführt wird, wenn eine Menge des lagenförmigen Elements, die einer einzelnen Einheit des absorbierenden Artikels entspricht, gefördert wird, wobei der getrennte Zuführprozess zwischen der Zuführposition und einer Position, in der die Pfadlänge geändert wird, durchgeführt wird.

4. Herstellungsverfahren des lagenförmigen Elements gemäß einem der Ansprüche 1 bis 3, wobei eine Position, an der die Pfadlänge geändert wird, näher an der Zusammenführungsposition als an der Zuführposition angeordnet ist.

5. Herstellungsverfahren des lagenförmigen Elements gemäß einem der Ansprüche 1 bis 4, das ferner einen Wendevorgang zum Wenden einer Förderrichtung der ersten Lage durch Wickeln der ersten Lage um ein stabförmiges Element mit einer axialen Richtung, die hinsichtlich einer zur Förderrichtung der ersten Lage senkrechten Richtung geneigt ist, umfasst, wobei die Zuführposition in Förderrichtung stromabwärts des stangenförmigen Elements angeordnet ist.

6. Herstellungsverfahren des lagenförmigen Elements gemäß Anspruch 5, das ferner einen getrennten Zuführungsprozess des Zuführens der ersten Lage in Richtung des stabförmigen Elements durch Steuerung der Zuführgeschwindigkeit der ersten Lage umfasst, so dass eine Menge der ersten Lage, die einer einzelnen Einheit des absorbierenden Artikels entspricht, jedes Mal zugeführt wird, wenn eine Menge des lagenförmigen Elements, die einer einzelnen Einheit des absorbierenden Artikels entspricht, gefördert wird, wobei der getrennte Zuführungsprozess an der Förderrichtung stromaufwärts des stabförmigen Elements durchgeführt wird.

7. Herstellungsverfahren des lagenförmigen Elements gemäß einem der Ansprüche 1 bis 6, wobei
beim Bereitstellungsprozess, wenn ein wartender zweiter Walzenkörper mit einem ersten Walzenkörper, der gerade bereitgestellt wird, verbunden wird, um eine erste Lage von dem zweiten Walzenkörper nach der Bereitstellung einer ersten Lage von dem ersten Walzenkörper bereitzustellen,
eine Position eines Startpunktes auf dem ersten Walzenkörper eines der saugfähigen Artikel mit einer Position eines Startpunktes auf dem zweiten Walzenkörper eines der saugfähigen Artikel ausgerichtet wird und die erste Lage des ersten Walzenkörpers und die erste Lage des zweiten Walzenkörpers miteinander verbunden werden.

8. Vorrichtung zur Herstellung eines lagenförmigen Elements durch Übereinanderlegen einer kontinuierlichen ersten Lage und einer kontinuierlichen zweiten Lage in einer Zusammenführungsposition, um das lagenförmige Element mit aneinander angrenzenden, absorbierenden Artikeln auszubilden, wobei die Herstellungsvorrichtung umfasst:
eine Bereitstellungseinheit, die zum Bereitstellen der ersten Lage, die Dehnbarkeit aufweist und periodisch mit einem Bild ausgebildet ist, von einem Walzenkörper ausgebildet ist;
eine Zuführeinheit, die zum Zuführen der ersten Lage in Richtung der Zusammenführungsposition ausgebildet ist, durch Steuerung einer Zuführgeschwindigkeit der ersten Lage, so dass eine Menge der ersten Lage, die einer einzelnen Einheit des absorbierenden Artikels entspricht, jedes Mal zugeführt wird, wenn eine Menge des lagenförmigen Elements, die einer einzelnen Einheit des absorbierenden Artikels entspricht, gefördert wird; und
eine Positionseinstellungseinheit, die zum Einstellen einer Position des Bildes hinsichtlich des absorbierenden Artikels ausgebildet ist, durch Erfassen des Bildes auf der ersten Lage und Ändern einer Pfadlänge von einer Zuführposition zu der Zusammenführungsposition basierend auf einem Ergebnis der Erfassung des Bildes, zwischen einer Zuführposition, in der die Zuführgeschwindigkeit der ersten Lage gesteuert wird, und der Zusammenführungsposition.

## Revendications

1. Procédé de fabrication d'un organe en forme de feuille en superposant une première feuille continue et une seconde feuille continue à une position de fusion pour configurer l'organe en forme de feuille ayant des articles absorbants agencés de façon contiguë, le procédé de fabrication comprenant :
un processus d'alimentation consistant à alimenter la première feuille ayant une extensibilité et formée périodiquement avec une image depuis un corps de rouleau ;
un processus d'amenée consistant à amener la première feuille vers la position de fusion en commandant une vitesse d'amenée de la première feuille de sorte qu'une quantité de la première feuille équivalente à une seule unité de l'article absorbant est amenée à chaque fois qu'une quantité de l'organe en forme de feuille équivalente à une seule unité de l'article absorbant est acheminée ; et
un processus de réglage de position consistant à régler une position de l'image par rapport à l'article absorbant en détectant l'image sur la première feuille, et en changeant une longueur de chemin depuis une position d'amenée où la vitesse d'amenée de la première feuille est commandée à la position de fusion d'après un résultat de détection de l'image.

2. Procédé de fabrication d'organe en forme de feuille selon la revendication 1, dans lequel
dans le processus d'amenée, la détection de l'image sur la première feuille, et la commande de la vitesse d'amenée de la première feuille d'après un résultat de détection de l'image.

3. Procédé de fabrication d'organe en forme de feuille selon la revendication 1 ou la revendication 2, comprenant en outre un processus d'amenée séparé consistant à amener la première feuille vers la position de fusion en commandant une vitesse d'amenée de la première feuille de sorte qu'une quantité de la première feuille équivalente à une seule unité de l'article absorbant est amenée à chaque fois qu'une quantité de l'organe en forme de feuille équivalente à une seule unité de l'article absorbant est acheminée, le processus d'amenée séparé étant réalisé entre la position d'amenée et une position où la longueur de chemin est changée.

4. Procédé de fabrication d'organe en forme de feuille selon l'une quelconque de la revendication 1 à la revendication 3, dans lequel une position où la longueur de chemin est changée est disposée plus près de la position de fusion que de la position d'amenée.

5. Procédé de fabrication d'organe en forme de feuille selon l'une quelconque de la revendication 1 à la revendication 4, comprenant en outre un processus de rotation consistant à faire tourner une direction d'acheminement de la première feuille en enveloppant la première feuille autour d'un organe en forme de barre ayant une direction axiale inclinée par rapport à une direction orthogonale à la direction d'acheminement de la première feuille, dans lequel
la position d'amenée est agencée au niveau du côté aval de direction d'acheminement de l'organe en forme de barre.

6. Procédé de fabrication d'organe en forme de feuille selon la revendication 5, comprenant en outre un processus d'amenée séparé consistant à amener la première feuille vers l'organe en forme de barre en commandant la vitesse d'amenée de la première feuille de sorte qu'une quantité de la première feuille équivalente à une seule unité de l'article absorbant est amenée à chaque fois qu'une quantité de l'organe en forme de feuille équivalente à une seule unité de l'article absorbant est acheminée, le processus d'amenée séparé étant réalisé au niveau du côté amont de direction d'acheminement de l'organe en forme de barre.

7. Procédé de fabrication d'organe en forme de feuille selon l'une quelconque de la revendications 1 à la revendication 6, dans lequel
dans le processus d'alimentation, lors de l'épissage d'un second corps de rouleau en attente sur un premier corps de rouleau qui est en cours d'alimentation de façon à alimenter une première feuille depuis le second corps de rouleau après l'alimentation d'une première feuille depuis le premier corps de rouleau,
une position d'un point de démarrage sur le premier corps de rouleau de l'un des articles absorbants est alignée avec une position d'un point de démarrage sur le second corps de rouleau de l'un des articles absorbants, et la première feuille du premier corps de rouleau et la première feuille du second corps de rouleau sont épissées ensemble.

8. Appareil pour fabriquer un organe en forme de feuille en superposant une première feuille continue et une seconde feuille continue à une position de fusion pour configurer l'organe en forme de feuille ayant des articles absorbants agencés de façon contiguë, l'appareil de fabrication comprenant :
une unité d'alimentation configurée pour alimenter la première feuille ayant une extensibilité et formée périodiquement avec une image, depuis un corps de rouleau ;
une unité d'amenée configurée pour amener la première feuille vers la position de fusion en commandant une vitesse d'amenée de la première feuille de sorte qu'une quantité de la première feuille équivalente à une seule unité de l'article absorbant est amenée à chaque fois qu'une quantité de l'organe en forme de feuille équivalente à une seule unité de l'article absorbant est acheminée ; et
une unité de réglage de position configurée pour régler une position de l'image par rapport à l'article absorbant en détectant l'image sur la première feuille et en changeant une longueur de chemin d'une position d'amenée à la position de fusion d'après un résultat de détection de l'image, entre une position d'amenée où la vitesse d'amenée de la première feuille est commandée et la position de fusion.
